(19)

**Europäisches Patentamt**

**European Patent Office**

**Office européen des brevets**

(11)  **EP 2 308 969 B1**

(12)  # EUROPÄISCHE PATENTSCHRIFT

<table>
<tr><td>(45) Veröffentlichungstag und Bekanntmachung des Hinweises auf die Patenterteilung:<br>**02.12.2015  Patentblatt 2015/49**</td><td>(51) Int Cl.:<br>**C12N 13/00** <sup>(2006.01)</sup>      **C12N 1/00** <sup>(2006.01)</sup></td></tr>
</table>

(21)  Anmeldenummer: **10009025.7**

(22)  Anmeldetag: **31.08.2010**

(54)  **Verfahren zur Beschleunigung der Zellproliferation**

Method for accelerating cell proliferation

Procédé d'accélération de la prolifération cellulaire

(84)  Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30)  Priorität: **29.09.2009   DE 102009043666**

(43)  Veröffentlichungstag der Anmeldung:
**13.04.2011   Patentblatt 2011/15**

(73)  Patentinhaber: **Karlsruher Institut für Technologie 76131 Karlsruhe (DE)**

(72)  Erfinder:
- **Frey, Wolfgang, Dr.**
  **76137 Karlsruhe (DE)**
- **Sträßner, Ralf**
  **76835 Gleisweiler (DE)**
- **Eing, Christian, Dr.**
  **76287 Rheinstetten (DE)**
- **Berghöfer, Thomas**
  **76356 Weingarten (DE)**
- **Gusbeth, Christian, Dr.**
  **76133 Karlsruhe (DE)**
- **Flickinger, Bianca**
  **76229 Karlsruhe (DE)**
- **Wüstner, Rüdiger**
  **76344 Eggenstein-Leopoldshafen (DE)**
- **Pacher, Michael**
  **76327 Pfinztal (DE)**

(56)  Entgegenhaltungen:
**WO-A2-2005/032646**

- **EING C ET AL: "Effects of nanosecond pulsed electric field exposure on arabidopsis thaliana" IEEE TRANSACTIONS ON DIELECTRICS AND ELECTRICAL INSULATION, IEEE SERVICE CENTER, PISCATAWAY, NJ, US LNKD- DOI: 10.1109/TDEI.2009.5293945, Bd. 16, Nr. 5, 1. Oktober 2009 (2009-10-01), Seiten 1322-1328, XP011278893 ISSN: 1070-9878**
- **GUSBETH C ET AL: "Evidences for membrane elektroporation during application of nanoseconds electrical pulses" PLASMA SCIENCE, 2004. ICOPS 2004. IEEE CONFERENCE RECORD - ABSTRACTS. THE 31ST IEEE INTERNATIONAL CONFERENCE ON BALTIMORE, MD, USA JUNE 28-JULY 1, 2004, PISCATAWAY, NJ, USA,IEEE LNKD- DOI: 10.1109/PLASMA.2004.1339774, 28. Juni 2004 (2004-06-28), Seiten 195-195, XP010728649 ISBN: 978-0-7803-8334-0**
- **SCARLETT S S ET AL: "Regulation of intracellular calcium concentration by nanosecond pulsed electric fields" BIOCHIMICA ET BIOPHYSICA ACTA. BIOMEMBRANES, AMSTERDAM, NL LNKD- DOI:10.1016/J.BBAMEM.2009.02.006, Bd. 1788, Nr. 5, 1. Mai 2009 (2009-05-01), Seiten 1168-1175, XP026023994 ISSN: 0005-2736 [gefunden am 2009-02-20]**
- **ZHANG JUE ET AL: "Nanosecond pulse electric field (nanopulse): A novel non-ligand agonist for platelet activation" ARCHIVES OF BIOCHEMISTRY AND BIOPHYSICS, Bd. 471, Nr. 2, März 2008 (2008-03), Seiten 240-248, XP002607060 ISSN: 0003-9861**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 308 969 B1

Printed by Jouve, 75001 PARIS (FR)                    **(Forts. nächste Seite)**

- BEEBE STEPHEN J ET AL: "Diverse effects of nanosecond pulsed electric fields on cells and tissues" DNA AND CELL BIOLOGY, MARY ANN LIEBERT, NEW YORK, NY, US LNKD- DOI: 10.1089/104454903322624993, Bd. 22, Nr. 12, 1. Dezember 2003 (2003-12-01), Seiten 785-796, XP002398892 ISSN: 1044-5498
- KOLB JUERGEN F ET AL: "Nanosecond pulsed electric field generators for the study of subcellular effects" BIOELECTROMAGNETICS, Bd. 27, Nr. 3, April 2006 (2006-04), Seiten 172-187, XP002607061 ISSN: 0197-8462
- WANG SUFEN ET AL: "Cardiac Myocyte Excitation by Ultrashort High-Field Pulses" BIOPHYSICAL JOURNAL, Bd. 96, Nr. 4, Februar 2009 (2009-02), Seiten 1640-1648, XP002607062 ISSN: 0006-3495
- SUNDARARAJAN RAJI: "Nanosecond Electroporation: Another Look" MOLECULAR BIOTECHNOLOGY, Bd. 41, Nr. 1, Januar 2009 (2009-01), Seiten 69-82, XP002607063 ISSN: 1073-6085
- BERGHOFER T ET AL: "Nanosecond electric pulses trigger actin responses in plant cells" BIOCHEMICAL AND BIOPHYSICAL RESEARCH COMMUNICATIONS, ACADEMIC PRESS INC. ORLANDO, FL, US, Bd. 387, Nr. 3, 25. September 2009 (2009-09-25), Seiten 590-595, XP026448324 ISSN: 0006-291X [gefunden am 2009-07-18]
- YEN-PATTON G P A ET AL: "ENDOTHELIAL CELL RESPONSE TO PULSED ELECTROMAGNETIC FIELDS STIMULATION OF GROWTH RATE AND ANGIOGENESIS IN-VITRO" JOURNAL OF CELLULAR PHYSIOLOGY, Bd. 134, Nr. 1, 1988, Seiten 37-46, XP002607064 ISSN: 0021-9541
- HILL JONATHAN ET AL: "Pulsed short-wave diathermy effects on human fibroblast proliferation." ARCHIVES OF PHYSICAL MEDICINE AND REHABILITATION JUN 2002 LNKD- PUBMED:12048663, Bd. 83, Nr. 6, Juni 2002 (2002-06), Seiten 832-836, XP002607065 ISSN: 0003-9993
- TAKAKI K ET AL: "Application of ies pulsed power generator for mushroom cultivation" PULSED POWER PLASMA SCIENCE CONFERENCE, 2007. PPPS 2007. IEEE, IEEE, PI, 17. Juni 2008 (2008-06-17), Seiten 1253-1256, XP031349745 ISBN: 978-1-4244-0913-6
- COSTANZO ET AL: "The influence of an electric field on the growth of soy seedlings" JOURNAL OF ELECTROSTATICS, ELSEVIER SCIENCE PUBLISHERS B.V. AMSTERDAM, NL LNKD- DOI: 10.1016/J.ELSTAT.2008.04.002, Bd. 66, Nr. 7-8, 1. Juli 2008 (2008-07-01), Seiten 417-420, XP022823725 ISSN: 0304-3886 [gefunden am 2008-05-21]
- TSUKAMOTO S ET AL: "Application of pulsed power to mushroom culturing" 14TH. INTERNATIONAL PULSED POWER CONFERENCE. PPC-2003. DIGEST OF TECHNICAL PAPERS. DALLAS, TX, JUNE 15 - 18, 2003; [IEEE PULSED POWER CONFERENCE], NEW YORK, NY : IEEE, US LNKD- DOI: 10.1109/PPC.2003.1278007, Bd. 2, 15. Juni 2003 (2003-06-15), Seiten 1116-1119, XP010690852 ISBN: 978-0-7803-7915-2
- BACHMAN C H ET AL: "SOME EFFECTS OF HIGH ELECTRICAL FIELDS ON BARLEY GROWTH" INTERNATIONAL JOURNAL OF BIOMETEOROLOGY, Bd. 17, Nr. 3, 1973, Seiten 253-262, XP002607066 ISSN: 0020-7128

**Beschreibung**

Die vorliegende Erfindung betrifft ein Verfahren zur Beschleunigung der Proliferation von Zellen, Pflanzen, Pilzen oder Geweben mit Hilfe eines gepulsten elektrischen Felds mit einer Dauer im Nanosekundenbereich.

Sowohl prokaryotische als auch eukaryotische Zellen sind von einer abgeschlossenen Plasmamembran umgeben, die das Zellinnere von der Umgebung abgrenzt. Die Plasmamembran besteht hauptsächlich aus einer elektrisch isolierenden Lipiddoppelschicht und darin eingelagerten Proteinen, die den Austausch von Molekülen oder Ionen zwischen dem Zellinneren und der Umgebung ermöglichen. Die Zellmembran kann durch Anlegen einer externen elektrischen Feldstärke aufgeladen werden.

Zum Beispiel beeinflussen gepulste elektrische Felder biologische Zellen, die in einer leitfähigen Flüssigkeit suspendiert wurden, derart, dass die elektrischen Impulse zur Ausbildung von Poren in der Plasmamembran führen. Die notwendige Membranaufladung, bei welcher eine Ausbildung von Membranporen beobachtet werden kann, liegt bei 0,5 bis 1 V. Man spricht hierbei von der sog. Elektroporation. Für die konventionelle Elektroporation werden elektrischen Impulse mit einer Spannung von 0,1 - 1 kV verwendet und die Pulsdauer ist mit mehr als 100 $\mu$s länger als die Zeit, die eine Plasmamembran benötigt, um die Aufladung von 0,5-1 V zu erreichen.

Die Zeit, die benötigt wird, um eine Plasmamembran aufzuladen hängt von verschiedenen elektrischen Parametern der Zelle und der Flüssigkeit, in welche die Zelle suspendiert wurde, ab. Für sphärische Zellen mit einer idealen dielektrischen Oberfläche gilt folgender Zusammenhang für die Zeitkonstante:

$$\tau_c = (\rho_c + \rho_a/2)\, c_m D/2$$

wobei $c_m$ die Kapazität der Plasmamembran pro Flächeneinheit, $\rho_c$ der spezifische Widerstand des Cytoplasmas, $\rho_a$ der spezifische Widerstand des Suspensionsmediums und D der Zelldurchmesser ist. Empirische Werte für den spezifischen Widerstand des Cytoplasmas sind bei ca. $\rho_c$ = 0,5-1 S/m und die Kapazität einer Plasmamembran ist in der Größenordnung $c_m$ = 1 $\mu$F/cm$^2$. Empirische Werte für die Zeitkostante liegen bei $\tau_c$ = 100 ns. Die Zeitkonstante gilt als Maß für die Zeit, während der das Zellinnere dem angelegten gepulsten elektrischen Feld ausgesetzt ist. Bei längeren Pulszeiten erfolgt eine Aufladung der Plasmamembran selbst und das elektrische Feld tritt nicht in das Zellinnere über.

Wird die Zelle externen elektrischen Feldern mit hohen Feldamplituden ausgesetzt und die Pulsdauer deutlich kürzer eingestellt, als die Zeit für die Membranaufladung, kann das elektrische Feld in das Zellinnere gelangen. Dieser Verschiebestrom kann intrazelluläre Membranen aufladen und die Zellorganellen beeinflussen.

Um einen Übertritt des gepulsten elektrischen Felds in das Innere der Zelle bzw. durch die Zelle hindurch zu gewährleisten, muss weiterhin die Anstiegsgeschwindigkeit des elektrischen Impulses schnell sein. D.h. die Spannung zwischen den Elektroden muss in möglichst kurzer Zeit ihr Maximum erreichen, damit sich die Plasmamembran nicht auflädt und die Äquipotentiallinien durch die Zellen hindurch gehen.

Akyiama *et al.* (S. Tsukamoto, T. Maeda, M. Ikeda and H. Akiyama, "Application of pulsed power to mushroom culturing", 14th IEEE Intern. Pulsed Power Conf., Vol. 2, pp. 1116-1119, 2003) berichten von einem Effekt, wonach die Ausbildung von Fruchtkörpern bei Ständerpilzen verstärkt auftritt, nachdem ein 1,2 m langer Baumstamm, der mit Shiitake Pilzen beimpft wurde, mit einer elektrischen Spannung von 140 kV und Pulsen von 200 ns Dauer behandelt wurde. Auch Takake *et al.* (K. Takaki, K. Kanesawa, N. Yamazaki, S. Mukaigawa, T. Fujiwara, K. Takahasi, K. Yamasita and K. Nagane, "Application of IES pulsed power generator for mushroom cultivation", 16th IEEE Intern. Pulsed Power Conf., Vol. 2, pp. 1253-1256, 2007) kommen zu vergleichbaren Ergebnissen mit dem Nameko Pilz. Wenn ein damit beimpfter Baustamm von 0,9 m Länge mit einer elektrischen Spannung von 120 kV und einer Pulsdauer von 100 ns behandelt wurde, konnte man eine 1,5 fach erhöhte Ausbildung von Fruchtkörpern beobachten. Diese Untersuchungen basieren auf der phänomenologischen Beobachtung, dass an Stellen, wo ein Blitzeinschlag stattgefunden hat, eine verstärkte Ausbildung von Pilzfruchtkörpern zu sehen war. Man geht davon aus, dass dieses Phänomen aufgrund der Entstehung von Bruchstellen oder Rissen in Pilzhyphen auftritt, da allgemein die Fruchtkörper von Ständerpilzen aus solchen Hyphenrissen entstehen. Ein wachstumsfördernder Effekt des gepulsten elektrischen Felds auf Pilzhyphen oder -myzelien wird hier nicht beschrieben.

Teilweise widersprüchliche Aussagen wurden bekannt bzgl. der wachstumsstimulierenden Effekte von elektrischen statischen oder langsamen Wechselfeldern (50/60 Hz) auf Pflanzenkeimlinge. Beispielsweise wurde berichtet, dass bei Sojakeimlingen in einem Wechselfeld von 50 Hz bei relativ niedriger Feldstärke von 36 V/cm die mittlere Sprosslänge um 12 % erhöht war, während bei einem statischen Feld derselben Stärke keinerlei Effekte zu beobachten waren (E. Costanzo, "The influence of an electric field on the growth of soy seedlings", J. Electrostatics, Vol. 66, pp. 417-420, 2008). Für Gerste hingegen konnte ein leicht verstärktes Wachstum nach einer fünftägigen Behandlung im elektrostatischen Feld von 0,5 kV/cm verzeichnet werden (C. H. Bachman and M. Reichmanis, "Some effects of high electrical fields on barley growth", Intern. J. Biometeorology, Vol. 17, pp. 253-262, 1973). Die Ergebnisse dieser Studien lassen keine allgemeinen Schlussfolgerungen zu, ob bzw. in wiefern elektrische Felder das Wachstum von Zellen, Pflanzen, Pilze oder Geweben beeinflussen, da die Resultate nicht signifikant (Wachstumssteigerung von 10%-20%) und teilweise widersprüchlich sind. Des Weiteren sind Wachstums fördernde Behandlungen im elektrischen Feld bei einem derart großen Energieeintrag wirtschaftlich nicht sinnvoll.

Der Einfluss von gepulsten elektrischen Feldern im Nanosekundenbereich auf die Proliferation von Zellen, Pflanzen, Pilzen oder Geweben wurde bislang nicht untersucht. Insbesondere sind keine Zellproliferation beschleunigenden Beobachtungen bei elektrischen Feldstärkeimpulsen mit sehr schnellen Impulsanstiegszeiten beschrieben.

Ausgehend hiervon ist es die Aufgabe der Erfindung, ein Verfahren zur Beschleunigung der Proliferation von Zellen, Pflanzen, Pilzen oder Geweben mittels eines gepulsten elektrischen Felds vorzuschlagen, welches zudem möglichst vielseitig anwendbar ist.

Die vorliegende Erfindung löst die Aufgabe durch das in Anspruch 1 beanspruchte Verfahren. Bevorzugte Ausführungsformen des Verfahrens sind in den rückbezogenen Ansprüchen beschrieben.

Die vorliegende Erfindung basiert darauf, dass biologisches Zellmaterial, entweder Einzeller oder Mehrzeller, in einer leitfähigen Flüssigkeit suspendiert werden und einem gepulsten elektrischen Feld von kurzer Dauer ausgesetzt werden. Die Zeit, mit der der Pulsanstieg des elektrischen Feldstärkeimpulses bis zum Erreichen von 10% bis 90% der Spannungsamplitude erfolgt, liegt zwischen 0,1 ns und 100 ns. Bevorzugter Weise liegt die Spannungsanstiegszeit zwischen 0,1 ns und 20 ns. Bei dem Verfahren kann auch eine Serie mehrerer elektrischer Impulse verwendet werden.

Im Rahmen der vorliegenden Erfindung umfasst der Begriff der Zellproliferation sowohl das Zellwachstum, also die Größen- und Volumenzunahme einzelner Zellen, als auch die Zellteilung.

Im Zusammenhang mit der vorliegenden Erfindung umfasst der Begriff des biologischen Zellmaterials sowohl eukaryontische als auch prokaryontische Zellen. Diese können als einzellige Lebewesen oder im Falle der Eukaryonten auch als mehrzellige Lebewesen für das erfindungsgemäße Verfahren vorliegen. Beispiele für einzelliges Zellmaterial sind Bakterien, Hefen oder Mikroalgen. Menschliche Stammzellen sind von diesem Verfahren ausgeschlossen. Mehrzelliges Zellmaterial umfasst Pflanzen und Pilze bzw. deren Sporen, Myzelien, Samen oder Keimlinge. Insbesondere umfasst die Erfindung Keimlinge von Pflanzen. Weiterhin bevorzugt werden Pilzsporen eingesetzt. Weiterhin besteht die Möglichkeit des Einsatzes von Geweben, die eine Ansammlung von Zellen darstellt, wie z.B. Meristeme bei Pflanzen sowie Epithelgewebe oder Bindegewebe bei Menschen oder Tieren.

Im erfindungsgemäßen Verfahren werden die Zellen vor der Behandlung mit einem gepulsten elektrischen Feld in einer leitfähigen Lösung suspendiert. Die elektrische Leitfähigkeit beträgt in einer bevorzugten Ausführung von 0,5 mS/cm bis 50 mS/cm, besonders bevorzugt von 1 mS/cm bis 10 mS/cm, insbesondere 4 mS/cm.

Die Dauer der Behandlung im gepulsten elektrischen Feld beträgt 5 ns bis 5000 ns, besonders bevorzugt ist der Bereich zwischen 10 ns und 100 ns. Allgemein erfasst die Impulsdauer die Zeit, während der die Zielspannung an den Elektroden anliegt. Die Dauer des elektrischen Feldstärkeimpulses beeinflusst die Effizienz des erfindungsgemäßen Verfahrens. Kurze Impulsdauern beeinflussen die Zellproliferation positiv. Abhängig vom vorliegenden Zellmaterial kann bei zu lange gewählten Impulsen auch das Wachstum gehemmt werden.

Die Anzahl der abgegebenen elektrischen Feldstärkeimpulse pro Behandlungszyklus hat einen Einfluss auf die Effizienz des erfindungsgemäßen Verfahrens. Je größer die Anzahl der Impulse desto höher ist die abgegebene Gesamtenergie. Abhängig vom Zellmaterial., der Leitfähigkeit der Suspension und der Impulsdauer ist die Anzahl der Feldstärkeimpulse optimierbar. Die Anzahl der elektrischen Impulse pro Behandlung beträgt zwischen 1 und 100.

Die Feldstärke des verwendeten elektrischen Feldstärkeimpulses beträgt zwischen 0,5 kV/cm und 50 kV/cm.

Aus dem Zusammenhang zwischen Pulsdauer, der Anzahl der Pulse sowie der angelegten Feldstärke ergibt sich der Gesamtenergieeintrag an elektrischer Energie, der pro Behandlungszyklus auf die Zellen abgegeben wird. Im Rahmen der angegebenen Intervalle können die Versuchsprotokolle jeweils optimiert werden, wobei aus ökonomischer Sicht ein möglichst minimaler Energieeintrag bei maximaler Zellproliferation zu bevorzugen ist. Bevorzugt werden Behandlungsenergien pro Behandlung von 10 J/kg (Zellsuspension) bis 10 kJ/kg (Zellsuspension).

Eine bevorzugte Ausführung ergibt sich bei der Verwendung von Zellkulturen, die in Flüssigmedien mittels sog. Fermenter angezogen werden. Dies betrifft insbesondere Mikroalgen, Bakterien oder Hefen, die zur Herstellung von Wirkstoffen, Lebensmitteln oder zur Herstellung von Energieträgern genutzt werden. Diese bereits in Suspension befindlichen Zellen können während ihrer Aufzucht durch eine entsprechende Elektrodenanordnung gefördert (gepumpt) werden. Dem Fachmann sind unterschiedlichste Elektrodenanordnungen und Formen aus dem Stand der Technik bekannt. Mit Hilfe der Elektroden werden wiederholt elektrische Impulse auf die Zellsuspension abgegeben. Somit kann die Behandlung der Zellen im elektrischen Feld im Dauerbetrieb stattfinden. Abhängig von Fermentervolumen, Durchflussgeschwindigkeit, und Zelldichte kann die Impulsfrequenz angepasst werden, sodass das im Fermenter befindliche Zellmaterial einen optimierten Energieeintrag erhält.

Die Erfindung wird im Folgenden mit Ausführungsbeispielen und folgenden Figuren erläutert.

**Fig. 1** Schematischer Versuchsaufbau eines Kabelimpulsgenerators.

**Fig. 2** Grafische Darstellung der mittleren Blattoberfläche **5** von *Arabidopsis thaliana* Keimlingen 5 Tage nach der Behandlung mit einem gepulsten elektrischen Feld mit unterschiedlichen Parametern.

**Fig. 3** Repräsentative Aufnahmen von Platten mit *Arabidopsis thaliana* Keimlingen auf MS-Agar 7 Tage nach der Behandlung mit unterschiedlichen Pulsparametern.

**Fig. 4** Aufnahmen von Myzelien des Ulmenraslings an verschiedenen Tagen nach der Behandlung der Sporen im gepulsten elektrischen Feld.

**Fig. 5** Grafische Darstellung der Fläche der Myzelien des Ulmenraslings als Funktion der Inkubationszeit.

**Beispiel 1:**

*Arabidopsis thaliana* Saat wurde Oberflächen sterilisiert, um eventuelle Kontaminationen zu entfernen. Hierzu wurde die Saat 15 Minuten bei Raumtemperatur in 4 % Natriumhypochloridlösung behandelt. Anschließend wurde die Saat durch Zentrifugieren (20 Sekunden bei 600 g) konzentriert und der Überstand unter keimfreien Bedingungen entfernt. Die Saat wurde fünfmal mit sterilisiertem Wasser gewaschen und anschließend in steriler Agaroselösung (1 % w/v) bei 4°C über zwei Tage gelagert. Die Saat wurde dann auf 90 mm Petrischalen mit Festmedium ausgesät und während 7 Tagen in einer Anzuchtkammer unter kontrollierten Bedingungen kultiviert.

Unmittelbar vor der Behandlung mit dem gepulsten elektrischen Feld wurde 10 Keimlinge sorgfältig von der Agarplatte abgelöst, in 4 mS/cm Behandlungs-Puffer (40 mM KCl, pH 6.5 mit TRIS/MES eingestellt) resuspendiert und in eine Elektroporationsküvette eingebracht. Jede Küvette wurde direkt vor der Pulsbehandlung gefüllt und mit gereinigtem, sterilem Paraffinöl überschichtet. Diese Maßnahme verhindert Überschläge an der Oberfläche bei hohen elektrischen Feldstärken. Nach der Behandlung mit dem gepulsten elektrischen Feld wurde das Paraffinöl mit einer Pipette entfernt und die Keimlinge aus der Küvette gespült, sorgfältig auf einer neuen Agarplatte ausgebracht und anschließend 11 Tage in einer Klimakammer kultiviert. Bei jedem Parametersatz wurden drei Behandlungen mit jeweils 10 Keimlingen durchgeführt. Weiterhin wurden als Kontrolle fünf Scheinbehandlungen mit identischem Vorgehen, jedoch ohne Applikation eines gepulsten elektrischen Feldes, durchgeführt.

Für alle Versuche wurde derselbe Typ Elektroporationsküvetten mit einem spezifizierten Elektrodenabstand von 4 mm und einem Füllvolumen von 800 $\mu$l verwendet.

Kabelimpulsgenerator:

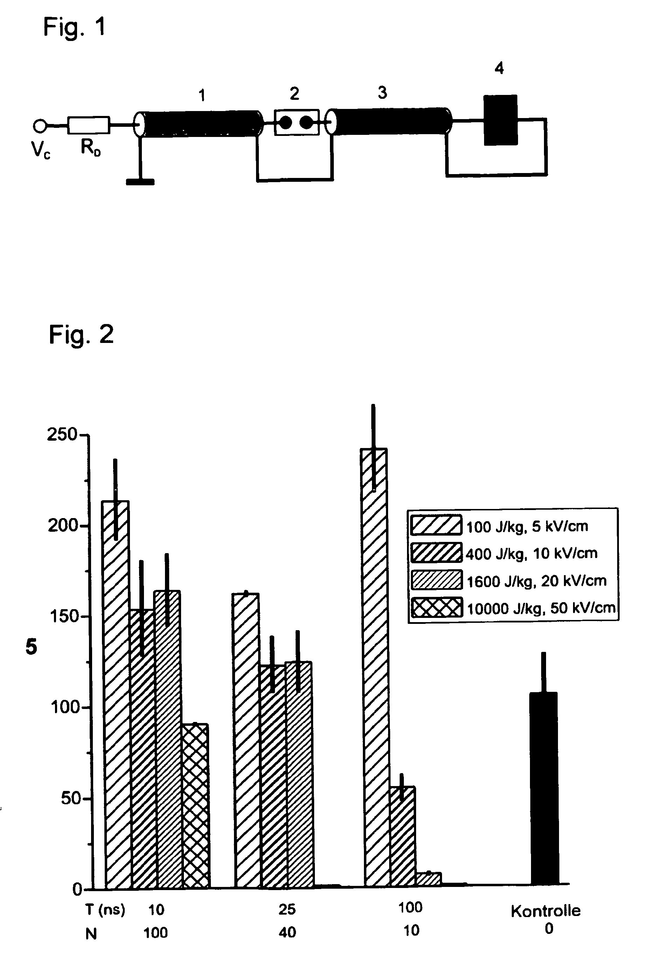

Die rechteckförmigen Pulse mit Spannungsamplituden von 2 kV, 4 kV, 8 kV und 20 kV und entsprechenden Feldstärken von 5 kV/cm, 10 kV/cm, 20 kV/cm und 50 kV/cm wurden mit Hilfe eines Kabelimpulsgenerators erzeugt, siehe Fig. 1. Handelsübliche 50 Ohm Koaxialkabel **1** wurden über einen Entkoppelwiderstand $R_D$ = 100 MOhm auf 40 kV aufgeladen und mit Hilfe einer $SF_6$-isolierten Funkenstrecke **2** über die Ausgangsleitung **3** auf das Versuchsgefäß **4** (Küvette) geschaltet. Die Pulslänge wurde über die Länge der pulsformenden Leitung **1** eingestellt. Bei den vorliegenden Experimenten betrugen die Kabellängen 1 m, 2.5 m und 10 m, was Pulslängen von 10 ns, 25 ns und 100 ns entspricht. Die Länge der Ausgangsleitung **3** des Generators betrug 1.2 m. Der Kabelimpulsgenerator wurde über ein 65 kV/8 mA Hochspannungsnetzteil geladen. Der zeitliche Verlauf der Ausgangsspannung wurde mittels eines 500 MHz Oszilloskops aufgezeichnet. Die Spannung wurde mit Hilfe eines kalibrierten Tektronix-Tastkopfs gemessen. Um die verwendbare Bandbreite des Tastkopfs zu erhöhen wurde das Plastikgehäuse des Tastkopfs entfernt, die Metallhülse des Tastkopfs massiv mit dem zentralen Massepunkt verbunden und ein 50 Ohm Dämpfungswiderstand zwischen dem Spannungsmesspunkt und der Hochspannungsspitze des Tastkopfs eingebaut. Zur Kalibrierung des Tastkopfs wurde ein Rechteckimpulsgenerator mit einer Pulsanstiegszeit von $t_R$ = 0.7 ns verwendet. Die Anstiegszeit des gesamten Datenerfassungssystems, also des mit dem Digitaloszilloskop verbundenen Tastkopfs, war geringer als $t_R$ = 2 ns. Um die Schalterimpedanz, welche sich vornehmlich auf die Pulsanstiegszeit auswirkt, zu minimieren, wurde die Funkenstrecke **2** in einem Druckbereich zwischen 0.8 MPa und 1.0 MPa betrieben. Die Grobeinstellung der Durchbruchspannung der Funkenstrecke **2** erfolgte über eine Variation des Abstandes zwischen den Funkenstreckenelektroden in einem Bereich von 0.1 mm bis 1.0 mm. Die Pulsanstiegszeit von 10 % auf 90% der Spannungsamplitude des Kabelimpulsgenerators betrug $t_R$ = 2.5 ns. Die Funkenstrecke **2** wurde im Selbstdurchbruch betrieben. Die absolute Abweichung der Impulsspannungsamplitude eines Impulses vom RMS-Wert war weniger als 3%. Der zeitliche Abstand während eines Experiments zwischen zwei Pulsen betrug 5 s. Bei jeder Feldstärke wurden 100 Pulse mit 10 ns Dauer, 40 Pulse mit 25 ns Dauer und 10 Pulse mit 100 ns Dauer als grundlegender Parametersatz verwendet, siehe Tabelle 1. Die an das Behandlungsgefäß gelieferte spezifische Energie, gegeben durch die applizierte Energie pro behandelter Masse, wurde über $W = N \cdot 0.5 \cdot C \cdot V_c^2 / 0.8 \cdot 10^{-3}$ kg berechnet, wobei N die Pulsanzahl, C die Kapazität der pulsformenden Leitung **1** (107 pF/m) und $V_c$ die Ladespannung der pulsformenden Leitung **1** bezeichnet. Die Temperatur und Leitfähigkeit der Pufferlösung wurden so eingestellt, dass sich für den Kabelimpulsgenerator eine angepasste Last ergibt. Während der Experimente konnten keine vom Behandlungsgefäß **4** verursachten Reflexionen beobachtet werden.

Bilder der Keimlinge wurden mit einer Digitalkamera aufgenommen. Die Bestimmung der Blattfläche erfolgte mit dem Programm ImageJ. Die angegebenen Werte für die Blattfläche sind Mittelwerte der gesamten Blattfläche dreier Agarplatten mit jeweils 10 Keimlingen.

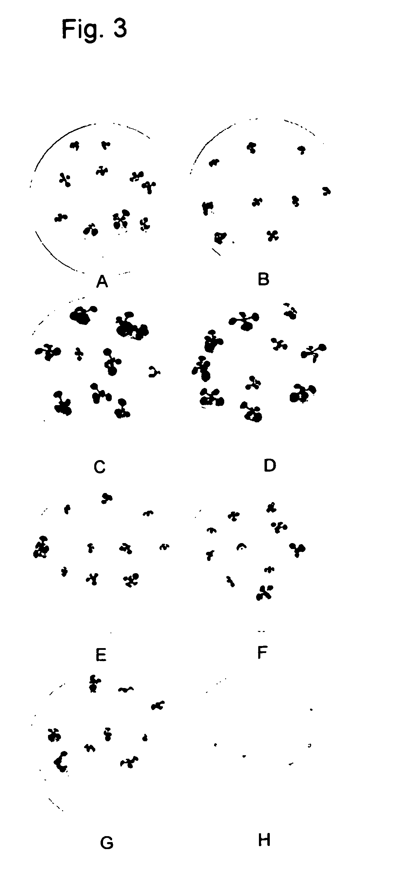

In **Figur 3 A-H** sind repräsentative Aufnahmen von *Arabidopsis thaliana* zu sehen, die nach der in Beispiel 1 beschriebenen Methode mit elektrischen Feldstärkeimpulsen behandelt wurden. Bereits bei geringem Energieeintrag wie in **Fig. 3 A,B** sind Kontrollkulturen, die nicht einer Behandlung mit einem elektrischen Feldstärkeimpuls unterzogen wurden. Die in **Fig. 3 C-H** abgebildeten Kulturen wurden mit den in der Tabelle 1 Parametern behandelt:

**Tabelle 1:**

| Fig. | 3 C | 3 D | 3 E | 3 F | 3 G | 3 H |
|---|---|---|---|---|---|---|
| Feldstärke [kV/cm] | 5 | 5 | 10 | 20 | 50 | 50 |
| Pulsdauer [ns] | 10 | 100 | 25 | 25 | 10 | 100 |
| Anzahl der Impulse | 100 | 10 | 40 | 40 | 100 | 10 |
| Behandlungsenergie[J/kg] | 100 | 100 | 400 | 1600 | 10000 | 10000 |

In **Fig. 3 C** und **D** ist eine im Vergleich zu den Kontrollkulturen **A** und **B** deutlich verstärkte Blattentwicklung zu erkennen, während ein höherer Energieeintrag nach den Parametern der **Fig. 3 E-G** nicht signifikant das Pflanzenwachstum beeinflusst. In **Fig. 3 H** ist ersichtlich, dass ein zu hoher Energieeintrag zu irreversiblen Schädigungen an der Pflanze führt.

Die Entwicklung der nach Beispiel 1 behandelten *Arabidopsis* Keimlinge mit unterschiedlichen Spannungen, Impulszeiten und Impulszahlen ist in **Fig. 2** graphisch dargestellt. Als Referenzwert für die Zellproliferation wurde hier die Blattoberfläche **5** gewählt. Im Vergleich zur unbehandelten Pflanze kann eine im Feldstärkeimpuls behandelte Pflanze bis zu 2,5 fach verstärkte Ausbildung der Blattoberfläche **5** erreichen.

**Beispiel 2:**

Die in Beispiel 1 beschriebene Vorrichtung wurde zur Behandlung von Pilzsporen des *Hypsizygus ulmaris* (Ulmenrasling) im gepulsten elektrischen Feld verwendet. Die Vorgehensweise war ähnlich des in Beispiel 1 beschriebenen Verfahrens, auch der Kabelimpulsgenerator war identisch. Um eine angepasste Last bei der Behandlung der Sporenlösung zu gewährleisten wurde diese mit einer 1 M KCl-Lösung auf eine Leitfähigkeit von 4 mS/cm eingestellt. Für die Behandlung mit dem gepulsten elektrischen Feld wurden 800 μl dieser Sporenlösung in eine Elektroporationsküvette mit einem Elektrodenabstand von 4 mm gefüllt und mit 100 μl sterilem entionisiertem Wasser überschichtet, um Oberflächenentladungen bei hohen Feldstärken zu vermeiden. Es wurden die in Tabelle 2 aufgelisteten Impulsparameter verwendet.

**Tabelle 2:**

| Feldstärke [kV/cm] | 5 | 10 | 20 | 20 | 50 |
|---|---|---|---|---|---|
| Pulsdauer [ns] | 100 | 25 | 25 | 100 | 25 |
| Anzahl der Impulse | 10 | 30 | 20 | 10 | 40 |
| Behandlungsenergie [mJ/ml] | 100 | 300 | 800 | 1600 | 10000 |

Nach der Pulsbehandlung wurden 100 μl der behandelten Sporenlösung entnommen und zum Animpfen einer Agarplatte verwendet. Die Agarplatten besitzen einen Durchmesser von 145 mm und enthielten ca. 100 ml antibiotischen Malzextraktagar.

Bei jedem Parametersatz wurden drei unabhängige Behandlungen durchgeführt. Zusätzlich wurden noch drei Scheinbehandlungen durchgeführt, bei dem die Sporenlösungen denselben Prozess durchliefen, jedoch ohne, dass sie einer Behandlung mit den gepulsten elektrischen Feldern unterzogen wurden.

Nach dem Animpfen wurden die Agarplatten in temperierten Aufzuchtboxen bei 25°C im Dunkeln inkubiert. Es wurde von jeder Agarplatte täglich ein Bild aufgenommen und die von Myzelien bewachsene Fläche wurde mit Hilfe des Programms ImageJ ausgewertet.

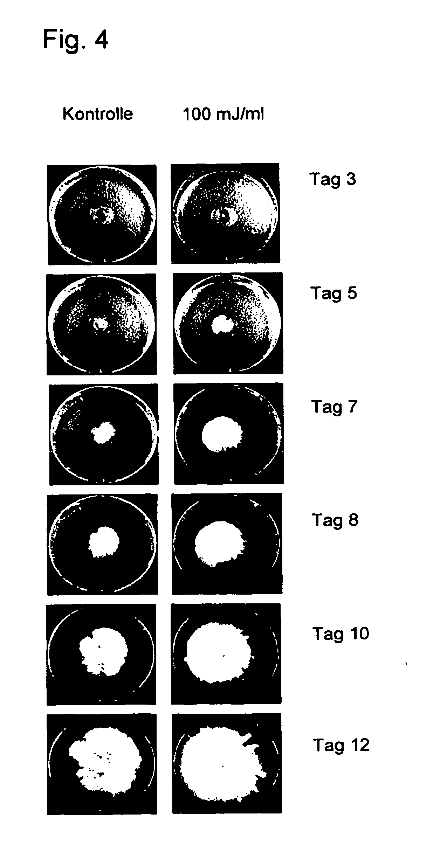

In **Fig. 4** sieht man die Aufnahmen der Pilzmyzelien auf Agarplatten nach verschieden Tagen der Inkubation der Sporen. Die rechte Spalte sind Myzelkulturen, die im gepulsten elektrischen Feld mit einer Feldstärke von 5 kV/cm, einer Pulsdauer von 100 ns und der Anzahl von 10 Impulsen behandelt wurden. Die linke Spalte ist im Vergleich hierzu die unbehandelte Kontrollkultur. Aus den Aufnahmen geht hervor, dass die Pilzmyzelien nach der Behandlung im gepulsten elektrischen Feld signifikant schneller entwickelt als bei der unbehandelten Kontrollkultur, was an der größeren Myzelfläche erkennbar ist. Beispielsweise bedeckt die behandelte Myzelkultur nach 6 Tagen eine um den Faktor 2,75 größere Fläche als die unbehandelte Kultur.

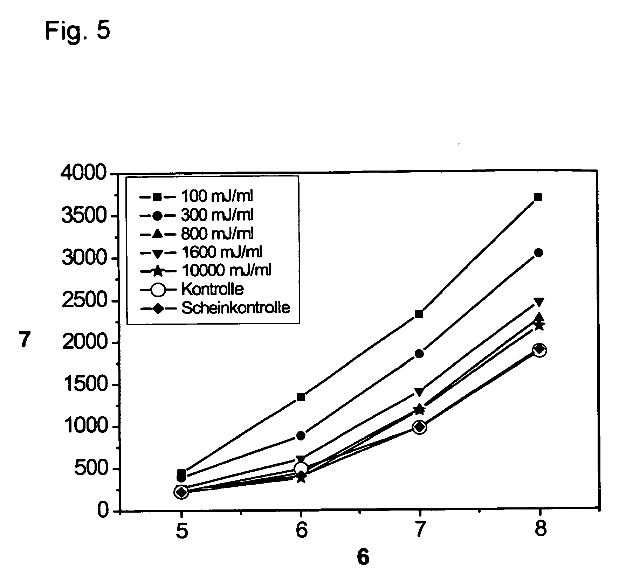

In **Fig. 5** ist die Entwicklung der unterschiedlich behandelten Sporen über den zeitlichen Verlauf von 5-8 Tagen nach der Behandlung im gepulsten elektrischen Feld grafisch dargestellt. Die jeweils eingesetzten Energieeinträge sind in der Grafik angegeben. Hier ist erkennbar, dass erstens die Stimulierung der Proliferation abhängig ist von der eingesetzten elektrischen Energie und zweitens, dass eine geringe Energiezufuhr ausreicht, um eine signifikant beschleunigte Proliferation des Pilzmyzels zu erreichen. Die höchste beobachtete beschleunigte Zellproliferation ist bereits mit 100 mJ/ml zu verzeichnen.

Demzufolge handelt es sich bei dem erfindungsgemäßen Verfahren um ein wirtschaftlich sinnvolles Verfahren, da bei relativ geringem Energieeintrag in Form von elektrischen Feldstärkeimpulsen eine deutlich erhöhte Ausbeute an Zellmaterial erhältlich ist. Insbesondere bei Zellkulturen in Fermentern, beispielsweise in der Insulinproduktion, sind kürzere Kulturzeiten mit erheblichen Einsparungen verbunden. Vorteilhaft ist eine beschleunigte Zellproliferation bei Gewebekulturen, wenn beispielsweise Patienten möglichst zügig Bedarf an *in vitro* kultiviertem Eigengewebe haben, z.B. Hautgewebe bei großflächigen Hautverbrennungen. Es ist ersichtlich, dass eine schnell wachsende *in vitro* Gewebekultur früher verpflanzt werden kann bei wesentlich reduzierter Morbidität der Patienten.

Abhängig von der Art der Kultur (z.B. Kultur auf festem oder flüssigem Medium), der Art kultivierten Zellen (Einzeller, Mehrzeller, Gewebe, Samen, Sporen) sowie den Kulturgefäßen kann die Elektrodenform entsprechend angepasst werden. Dem Fachmann ist eine Vielzahl von Elektrodenarten aus dem Stand der Technik bekannt.

**Bezugzeichenliste**

1    Pulsformende Leitung

2    Funkenstrecke

3    Ausgangsleitung

4    Versuchsgefäß

5    Blattoberfläche (mm$^2$)

6    Tage nach Behandlung

7    Gemittelte Myzeloberfläche (mm$^2$)

**Patentansprüche**

1. Verfahren zur Beschleunigung der Zellproliferation von biologischem Zellmaterial, umfassend folgende Verfahrensschritte:

   a. Bereitstellen eines Zellmaterials und suspendieren des Zellmaterials in einer elektrisch leitfähigen Flüssigkeit zu einer Suspension, die zwischen zwei Elektroden positioniert ist oder wird,
   b. Abgabe von 1 bis 100 elektrischen Feldstärkeimpulsen zwischen den Elektroden,

   **dadurch gekennzeichnet, dass** die elektrischen Feldstärkeimpulse derart abgegeben werden, dass ein Spannungsanstieg zwischen den beiden Elektroden von 10 % bis 90 % einer Zielspannung der elektrischen Feldstärkeimpulse innerhalb einer Zeitdauer von 0,1 bis 100 ns erfolgt, die elektrischen Feldstärkeimpulse eine Impulsdauer von 5 ns bis 5000 ns aufweisen, und dass die elektrischen Feldstärkeimpulse bei Erreichen der Zielspannung eine elektrische Feldstärke von 0,5 kV/cm bis 50 kV/cm aufweisen.

2. Verfahren nach Anspruch 1, wobei der Spannungsanstieg des mindestens einen elektrischen Feldstärkeimpulses innerhalb einer Zeitdauer von 0,1 bis 20 ns erfolgt.

3. Verfahren nach Anspruch 2, wobei der mindestens eine elektrische Feldstärkeimpuls eine Impulsdauer zwischen 10 ns und 100 ns aufweist.

4. Verfahren nach einem der vorgenannten Ansprüche, wobei mittels des mindestens einen elektrischen Feldstärkeimpulses eine Behandlungsenergie auf das biologische Zellmaterial eingebracht wird, deren Betrag von 10 J/kg(Zellsuspension) bis 10 kJ/kg(Zellsuspension) liegt.

5. Verfahren nach einem der vorgenannten Ansprüche, wobei das biologische Zellmaterial mehrzellige Lebewesen oder Gewebe von mehrzelligen Lebewesen enthält.

6. Verfahren nach Anspruch 5, wobei das biologische Zellmaterial Pflanzensamen oder -keimlinge enthält.

7. Verfahren nach Anspruch 5, wobei das biologische Zellmaterial Pilzsporen oder -myzelien enthält.

8. Verfahren nach einem der vorgenannten Ansprüche, wobei das biologische Zellmaterial einzellige Lebewesen enthält.

9. Verfahren nach Anspruch 8, wobei das biologische Zellmaterial Mikroalgen enthält.

10. Verfahren nach einem der vorgenannten Ansprüche, wobei auf die Suspension mindestens ein elektrischer Feldstärkeimpuls in einem Durchflussverfahren abgegeben wird.

**Claims**

1. Method for accelerating cell proliferation of biological cell material, comprising the following method steps:

   a. provision of a cell material, and suspending the cell material in an electrically conductive fluid to a suspension which is or will be positioned between two electrodes,
   b. emission of 1 to 100 electrical field strength pulses between the electrodes,

   **characterised in that** the electrical field strength pulses are emitted in such a way that a voltage rise between the two electrodes of 10 % to 90 % of a target voltage of the electrical field strength pulses occurs within a time duration from 0.1 to 100 ns, the electrical field strength pulses exhibit a pulse duration from 5 ns to 5000 ns, and that the electrical field strength pulses, on reaching the target voltage, exhibit an electrical field strength of 0.5 kV/cm to 50 kV/cm.

2. Method according to claim 1, wherein the voltage rise of the at least one electrical field strength pulse takes place within a time duration from 0.1 to 20 ns.

3. Method according to claim 2, wherein the at least one electrical field strength pulse exhibits a pulse duration of between 10 ns and 100 ns.

4. Method according to any one of the preceding claims, wherein, by means of the at least one electrical field strength pulse, a treatment energy is applied onto the biological cell material, the amount of which lies between 10 J/kg (cell suspension) to 10 kJ/kg (cell suspension).

5. Method according to any one of the preceding claims, wherein the biological cell material contains multi-cellular living organisms or contain tissue from multi-cellular living organisms.

6. Method according to claim 5, wherein the biological cell material contains plant seeds or seedlings.

7. Method according to claim 5, wherein the biological cell material contains mushroom spores or mycelia.

8. Method according to any one of the preceding claims, wherein the biological cell material contains single-cell living organisms.

**9.** Method according to claim 8, wherein the biological cell material contains microalgae.

**10.** Method according to any one of the preceding claims, wherein at least one electrical field strength pulse is emitted onto the suspension in a flow-through process.

**Revendications**

**1.** Procédé permettant d'accélérer la prolifération cellulaire dans un matériau cellulaire biologique comprenant les étapes consistant à :

a. préparer un matériau cellulaire et mettre en suspension ce matériau cellulaire dans un liquide électriquement conducteur pour obtenir une suspension qui est ou sera positionnée entre deux électrodes,
b. appliquer 1 à 100 impulsions d'intensité de champ électriques entre les électrodes,

**caractérisé en ce que**
les impulsions d'intensité de champ électriques sont appliquées de façon à obtenir une augmentation de la tension entre les deux électrodes de 10% à 90 % d'une tension cible des impulsions d'intensité de champ électriques dans un laps de temps de 0,1 à 100 ns, les impulsions d'intensité de champ électriques ont une durée d'impulsion de 5 ns à 5000 ns, et les impulsions d'intensité de champ électriques présentent lors de l'atteinte de la tension cible une intensité de champ électrique de 0,5 kV/cm à 50 kV/cm.

**2.** Procédé conforme à la revendication 1, selon lequel l'augmentation de tension de l'impulsion d'intensité de champ électrique se produit dans un laps de temps de 0,1 à 20 ns.

**3.** Procédé conforme à la revendication 2, selon lequel l'impulsion d'intensité de champ électrique a une durée d'impulsion comprise entre 10 ns et 100 ns.

**4.** Procédé conforme à l'une des revendications précédentes, selon lequel l'impulsion d'intensité de champ électrique applique sur le matériau cellulaire biologique une énergie de traitement dont la valeur est située entre 10 J/kg (suspension cellulaire) à 10 kJ/kg (suspension cellulaire).

**5.** Procédé conforme à l'une des revendications précédentes, selon lequel le matériau cellulaire biologique renferme des organismes multicellulaires ou des tissus d'organismes multicellulaires.

**6.** Procédé conforme à la revendication 5, selon lequel le matériau cellulaire renferme des semences ou des germes végétaux.

**7.** Procédé conforme à la revendication 5, selon lequel le matériau polymère renferme des spores ou des micelles de champignons.

**8.** Procédé conforme à l'une des revendications précédentes, selon lequel le matériau cellulaire biologique renferme des organismes monocellulaires.

**9.** Procédé conforme à la revendication 8, selon lequel le matériau cellulaire biologique renferme des micro-algues.

**10.** Procédé conforme à l'une des revendications précédentes, selon lequel au moins une impulsion d'intensité de champ électrique est appliquée sur la suspension selon un procédé de circulation.

## Fig. 1

## Fig. 2

# Fig. 3

A

B

C

D

E

F

G

H

# Fig. 4

Kontrolle 100 mJ/ml

Tag 3

Tag 5

Tag 7

Tag 8

Tag 10

Tag 12

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **S. TSUKAMOTO ; T. MAEDA ; M. IKEDA ; H. AKIYAMA.** Application of pulsed power to mushroom culturing. *14th IEEE Intern. Pulsed Power Conf.,* 2003, vol. 2, 1116-1119
- **K. TAKAKI ; K. KANESAWA ; N. YAMAZAKI ; S. MUKAIGAWA ; T. FUJIWARA ; K. TAKAHASI ; K. YAMASITA ; K. NAGANE.** Application of IES pulsed power generator for mushroom cultivation. *16th IEEE Intern. Pulsed Power Conf.,* 2007, vol. 2, 1253-1256

- **E. COSTANZO.** The influence of an electric field on the growth of soy seedlings. *J. Electrostatics,* 2008, vol. 66, 417-420
- **C. H. BACHMAN ; M. REICHMANIS.** Some effects of high electrical fields on barley growth. *Intern. J. Biometeorology,* 1973, vol. 17, 253-262